# EUROPEAN PATENT APPLICATION

(11) **EP 3 167 793 A1**
(43) Date of publication of application: **17.05.2017**
(21) Application number: 15193986.5
(22) Date of filing: 10.11.2015
(51) Int. Cl.: A61B 5/00, A61B 5/11

(54) **ELASTIC PHYSIOLOGICAL DETECTION STRUCTURE**

(71) Applicant: King's Metal Fiber Technologies Co., Ltd., 42060 Taichung City (TW)
(72) Inventor: KANG, Yu Hsun, 10451 Taipei City (TW); CHANG, Li Chuan, 10451 Taipei City (TW); HOU, Jaang Jiun, 10451 Taipei City (TW); WANG, Hao Chen, 10451 Taipei City (TW); LIAO, Shu Fen, 10451 Taipei City (TW); CHEN, Reng Sho, 10451 Taipei City (TW)
(74) Representative: Cabinet Chaillot

(57) **Abstract**

An elastic physiological detection structure includes a fabric article, a main body, an elastic member, and a detection module. The main body is combined with an internal layer of the fabric article. The main body includes at least one opening formed therein. The elastic member is combined to a surface of the main body. The detection module is combined to the elastic member and exposed outside the opening of the main body. The elastic member helps the detection module to apply an increased force from the fabric article to a surface layer of a human body, allowing the detection module located outside the opening of the main body to tightly engage the surface layer of the human body at a site where detection is to be made thereby improving the attachability of the detection module to the surface layer of the human body and enhancing stability of dynamic detection.

## Description

### BACKGROUND OF THE INVENTION

### 1. Field of the Invention

The present invention relates to an elastic physiological detection structure, and more particularly to one that comprises a combination of a fabric article, a main body, an elastic member, and a detection module, in which the elastic member helps the detection module to apply an increased force from the fabric article to the surface layer of the human body to improve the attachability of the detection module to the surface layer of the human body and make it applicable to a physiological detective garment, an inclination detective garment, or the likes..

### 2. Description of Related Art

The progress of science and technology makes it possible to develop a combination of detection modules with clothing in order to facilitate inspect and record the physiological conditions of a human body and allows for applications to self-management of home healthcare or preventive medical treatments.

Heretofore, the combination of a detection module with clothing is generally fixed. In other words, the detection module is retained, through sewing, at a location, such as chest, where detection is to be made in order to allow the detection module to engage a skin surface of a human body for detection of physiological signals of the human body.

However, the conventional way of combination through a fixed manner would result in signal interruption due to wearing of the clothing, so that the detection of physiological signals may be unexpectedly interrupted and then resumes. The continuity of the detected physiological signal could be destroyed so that the detection has to be conducted again. This causes undesired burdens of a user.

Thus, in view of the above problems, the present invention aims to provide an elastic physiological detection structure that effectively improves stability of dynamic detection and allows for easy operation and installation by a user.

### SUMMARY OF THE INVENTION

The primary object of the present invention is to provide an elastic physiological detection structure, which comprises a combination of a fabric article, a main body, an elastic member, and a detection module. The main body is combined with an internal layer of the fabric article. The main body comprises at least one opening formed therein. The elastic member is combined with a surface of the main body. The detection module is combined with the elastic member and exposed outside the opening of the main body. Thus, the elastic member helps the detection module to apply an increased force from the fabric article to the surface layer of the human body, allowing the detection module that is located outside the opening of the main body to tightly engage the surface layer of the human body at a site where detection is to be made thereby improving the attachability of the detection module to the surface layer of the human body and enhancing stability of dynamic detection, and thus improving overall utilization.

A secondary object of the present invention is to provides an elastic physiological detection structure, in which the opening of the main body has a predetermined length and the elastic member comprises a predetermined preserved length, so that the detection module is allowed to displace in the opening of the main body by means of the preserved length of the elastic member, allowing the detection module to move back and forth in the opening of the main body to provide an effect of changing the site where detection is to be made for detecting physiological signals of different sites of the human body. Further, with such an arrangement, the stability of the detection module can be ensured and the phenomenon of inflaming caused by the detection module being retained on and depressing a fixed location of the skin, which may lead to discomfort, can be eliminated. Thus, overall utilization can be improved.

A further object of the present invention is to provide an elastic physiological detection structure, in which the elastic member comprises an extension section and the extension section extends a sewing line of the fabric article to help keep the detection module that is combined with the elastic member and exposed outside the opening of the main body in a flat form and is bonded to the surface layer of the human body by the elastic member to allow the detection module that is exposed outside the opening of the main body to attach to the skin at a site where detection is to be made so as to improve the overall utilization.

To achieve above objects, the present invention provides an elastic physiological detection structure, which comprises: a fabric article, a main body, an elastic member, and a detection module. The main body is combined with an internal layer of the fabric article. The main body comprises at least one opening formed therein. The elastic member is combined with a surface of the main body. The detection module is combined with the elastic member and exposed outside the opening of the main body so that the detection module may apply an increased force by means of the elastic member.

### BRIEF DESCRIPTION OF THE DRAWINGS

The present invention can be fully understood from the following detailed description and preferred embodiments with reference to the accompanying drawings, in which:
Figure 1 is a perspective view shows a first embodiment of the present invention;
Figure 2 is a perspective view illustrating displacement of a detection module of the first embodiment of the present invention;
Figure 3 is a perspective view shows a second embodiment of the present invention;
Figure 4 is an exploded view of the second embodiment of the present invention;
Figure 5 is a perspective view illustrating displacement of a detection module of the second embodiment of the present invention;
Figure 6 is a perspective view showing a film layer coupled to an inside surface of an opening of the second embodiment of the present invention;
Figure 7 is a perspective view shows a third embodiment of the present invention;
Figure 8 is a schematic view showing an extension section of an elastic member of a fourth embodiment of the present invention combined with a single sewing line of a fabric article;
Figure 9 is a schematic view showing the extension section of the elastic member of the fourth embodiment of the present invention combined with two sewing lines of a fabric article;
Figure 10 is a schematic view showing the extension section of the elastic member of the fourth embodiment of the present invention extending a complete circumference around a fabric article;
Figure 11 is a perspective view showing a film layer coupled to an inside surface of an opening of the fourth embodiment of the present invention; and
Figure 12 is a schematic view illustrating two detection modules included in the elastic member of the fourth embodiment of the present invention.

### DETAILED DESCRIPTION OF THE PREFERRED EMBODIMENTS

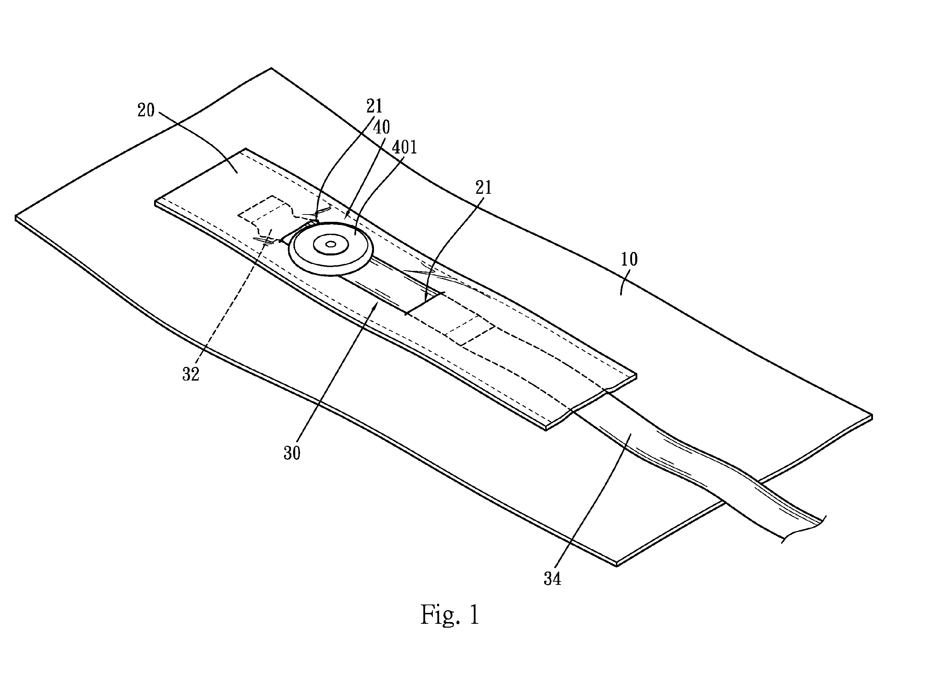

Referring to Figures 1-12, embodiments of the present invention are shown, in which a preferred form of an elastic physiological detection structure according to the present invention is applicable to detection of variation of a physiological signal of a surface layer of a human body or an inclination angle of a human body and also provides an effect of improving stability of dynamic detection and enhance attachability to the surface layer of the human body and thus improving overall utilization.

A first embodiment of the elastic physiological detection structure according to the present invention comprises a fabric article 10, a main body 20, an elastic member 30, and a detection module 40. The main body 20 is combined with an internal layer of the fabric article 10 and the combination is achieved with one of sewing, ultrasonic means, thermal fusion, and adhesive. The fabric article 10 can be one of a garment, trousers, gloves, underwear, a vest, a corsage, and a tube-top. (In the instant embodiment, a large piece of fabric that has not been tailored and processed to form the above listed forms of clothing is taken as an example for the fabric article.) Further, the main body 20 comprises at least one opening 21 formed therein. The opening 21 may extend transversely or longitudinally. (In the instant embodiment, an example that includes two longitudinal openings 21 is illustrated, as shown in Figure 1.) The main body 20 can be one of a piece of fabric, a woven article, and a membrane. (In the instant embodiment, a piece of fabric is used as an example for the present invention.) The membrane can be a plastic membrane, a metal membrane, a carbon membrane, or a membrane made of other materials.

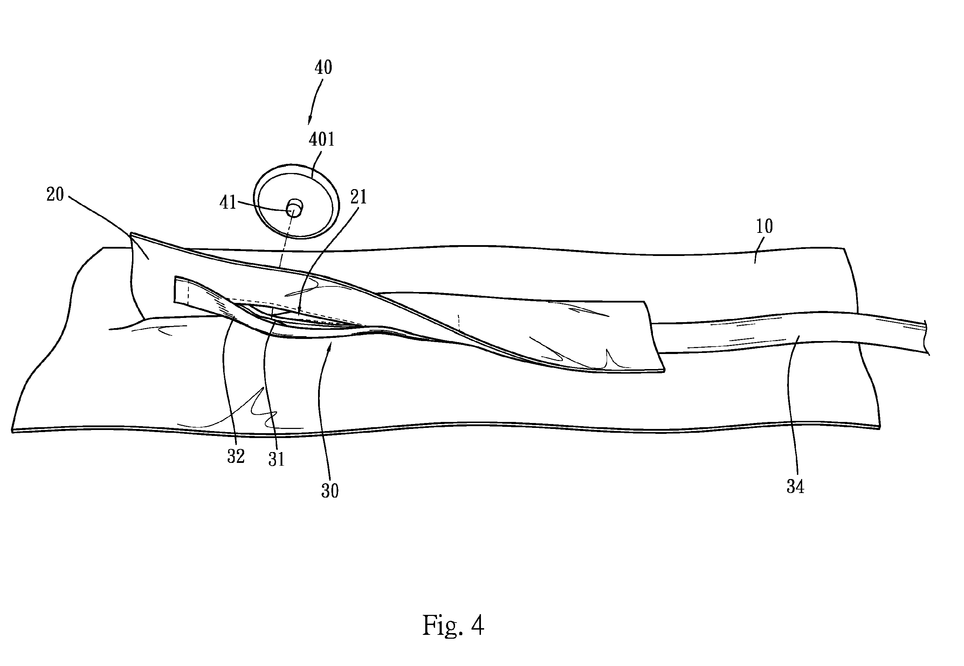

Further, the elastic member 30 is combined to a surface of the main body 20 and the combination is achieved with one of sewing, ultrasonic means, thermal fusion, and adhesive, or alternatively, two ends of the elastic member 30 and the main body 20 are respectively provided with engageable fastening sections (such as buttons, snaps, hook and loop fasteners, and the likes). (In the instant embodiment, sewing is taken as an example.) Further, the detection module 40 is combined to the elastic member 30 and the combination is achieved by providing the detection module 40 and the elastic member 30 respectively with mateable coupling sections 31, 41 (such as snaps, female-male pairs, and the likes), see Figure 4, or alternatively, the detection module 40 is directly mounted to the elastic member 30 (by means of for example one of sewing, ultrasonic means, thermal fusion, and adhesive). The detection module 40 is exposed outside the opening 21 of the main body 20. (In the instant embodiment, the elastic member 30 is set through the two longitudinal openings 21 of the main body 20 to allow the detection module 40 to be located outside and between the two longitudinal openings 21 and thus exposed.) The detection module 40 comprises a contact face and the contact face of the detection module 40 is provided and arranged to contact a surface layer of a human body in order to detect a physiological signal of the human body. In this way, the elastic member 30 helps the detection module 40 to apply an increased force from the fabric article 10 to the surface layer of the human body, allowing the detection module 40 that is located outside the opening 21 of the main body 20 to tightly engage the surface layer of the human body at a site where detection is to be made thereby improving the attachability of the detection module 40 to the surface layer of the human body.

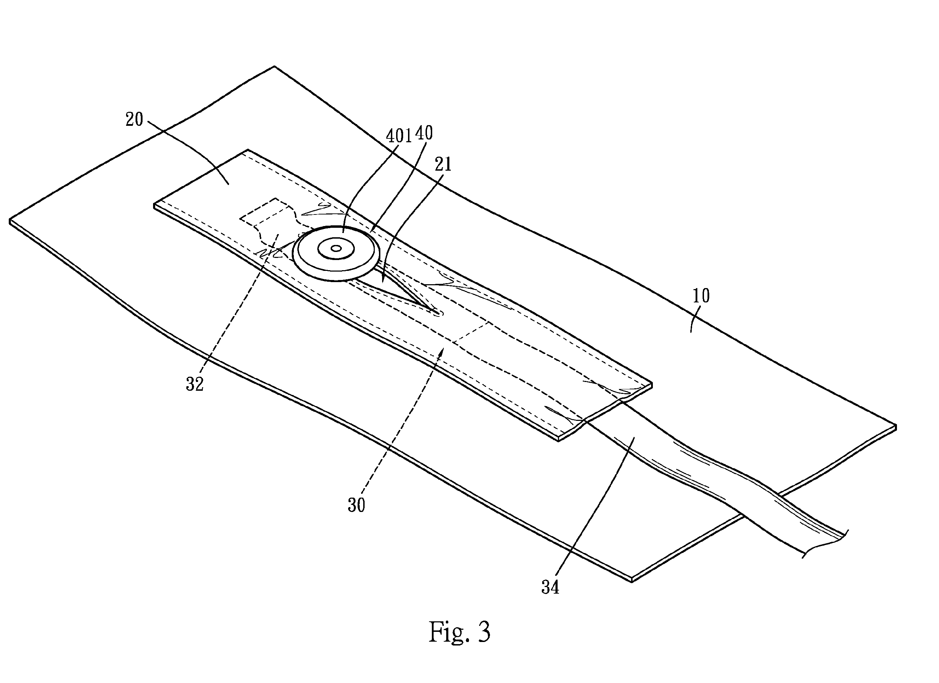

A second embodiment of the elastic physiological detection structure according to the present invention comprises a fabric article 10, a main body 20, an elastic member 30, and a detection module 40. The main body 20 is combined with an internal layer of the fabric article 10 and the combination is achieved with one of sewing, ultrasonic means, thermal fusion, and adhesive. The fabric article 10 can be one of a garment, trousers, gloves, underwear, a vest, a corsage, and a tube-top. (In the instant embodiment, a large piece of fabric that has not been tailored and processed to form the above listed forms of clothing is taken as an example for the fabric article.) Further, the main body 20 comprises at least one opening 21 formed therein. The opening 21 may extend transversely or longitudinally. (In the instant embodiment, an example that includes one transverse opening 21 is illustrated, as shown in Figure 3.) The main body 20 can be one of a piece of fabric, a woven article, and a membrane. (In the instant embodiment, a piece of fabric is used as an example for the present invention.) The membrane can be a plastic membrane, a metal membrane, a carbon membrane, or a membrane made of other materials.

Further, the elastic member 30 is combined to a surface of the main body 20 and the combination is achieved with one of sewing, ultrasonic means, thermal fusion, and adhesive, or alternatively, two ends of the elastic member 30 and the main body 20 are respectively provided with engageable fastening sections (such as buttons, snaps, hook and loop fasteners, and the likes). (In the instant embodiment, sewing is taken as an example.) Further, the detection module 40 is combined to the elastic member 30 and the combination is achieved by providing the detection module 40 and the elastic member 30 respectively with mateable coupling sections 31, 41 (such as snaps, female-male pairs, and the likes), see Figure 4, or alternatively, the detection module 40 is directly mounted to the elastic member 30 (by means of for example one of sewing, ultrasonic means, thermal fusion, and adhesive). The detection module 40 is exposed outside the opening 21 of the main body 20. (In the instant embodiment, the detection module 40 is located outside the transverse opening 21 and is thus exposed.) The detection module 40 comprises a contact face and the contact face of the detection module 40 is provided and arranged to contact a surface layer of a human body in order to detect a physiological signal of the human body. In this way, the elastic member 30 helps the detection module 40 to apply an increased force from the fabric article 10 to the surface layer of the human body, allowing the detection module 40 that is located outside the opening 21 of the main body 20 to tightly engage the surface layer of the human body at a site where detection is to be made thereby improving the attachability of the detection module 40 to the surface layer of the human body.

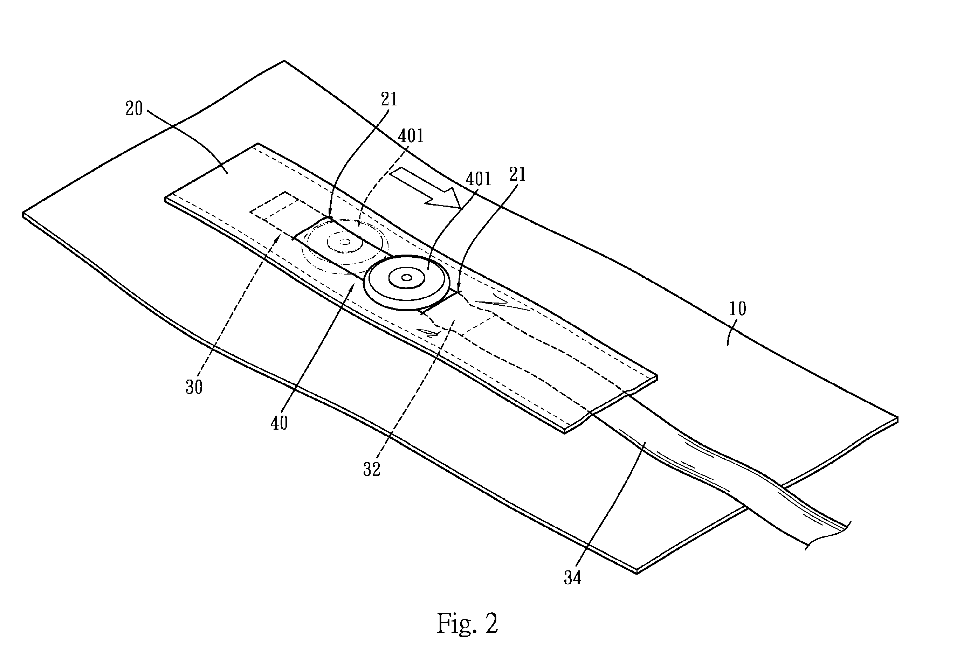

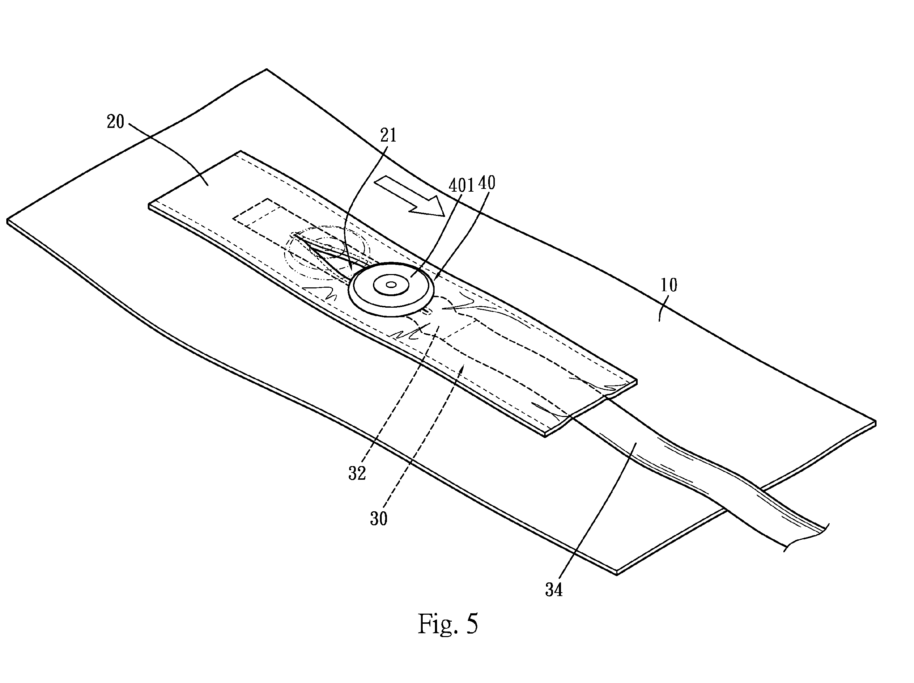

In the above-described first and second embodiments of the elastic physiological detection structure according to the present invention, the opening 21 of the main body 20 may be constructed to have a predetermined length. (These embodiments are respectively constructed to have two longitudinal openings 21 and one transverse opening 21.) And, the elastic member 30 has a predetermined preserved length 32 (as shown in Figures 1 and 3). The detection module 40 comprises a contact face and the contact face of the detection module 40 is provided for contacting the surface layer of the human body in order to detect the physiological signal of the surface layer of the human body. The detection module 40 is arranged to displace, through sliding, by means of the preserved length 32 of the elastic member 30 located inside the main body 20 (as shown in Figure 2 and 5), so as to all the detection module 40 to provide an effect of changing the detection site within the range of or between the opening(s) 21 of the main body 20.

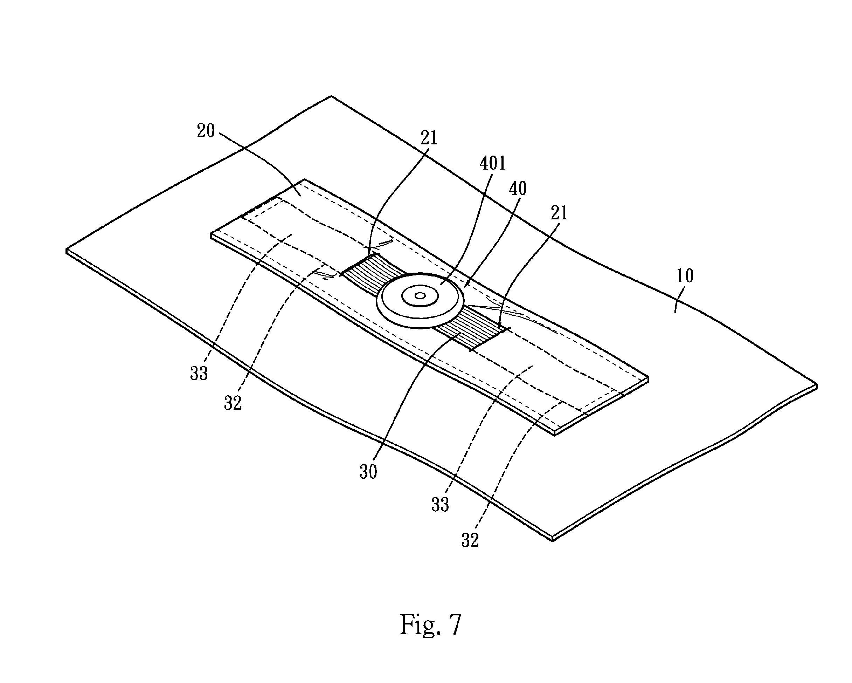

A third embodiment of the elastic physiological detection structure according to the present invention comprises a fabric article 10, a main body 20, an elastic member 30, and a detection module 40 and has a structural arrangement that is similar to those of the first and second embodiments with a primary difference being that the preserved length 32 of the elastic member 30 of the first and second embodiments is provided with at least one non-elastic section 33 (as shown in Figure 7). In the embodiment, an example that includes two longitudinal openings 21 is presented. (However, the present invention is not limited to such an example, and is also applicable to an example including one transverse opening 21.) Alternatively, the non-elastic section 33 can be provided at each of two ends of the elastic member 30 at two sides of the detection module 40 and the detection module 40 is mounted to a middle portion of the elastic member 30 that has elasticity. The non-elastic section 33 may be combined with the elastic member 30 during the weaving or knitting thereof or, alternatively, any suitable means of combination may be adopted to combine the non-elastic section 33 and the elastic member 30 together. The combination may be one of sewing, ultrasonic means, thermal fusion, and adhesive. The non-elastic sections 33 of the two ends of the elastic member 30 that are arranged at two sides of the detection module 40 allow the detection module 40 to displace easily.

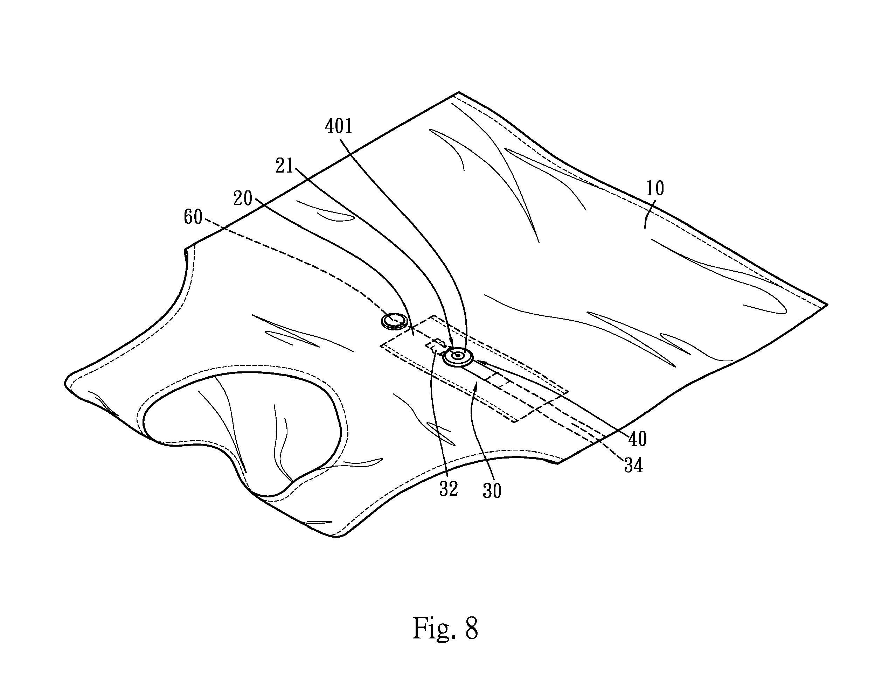

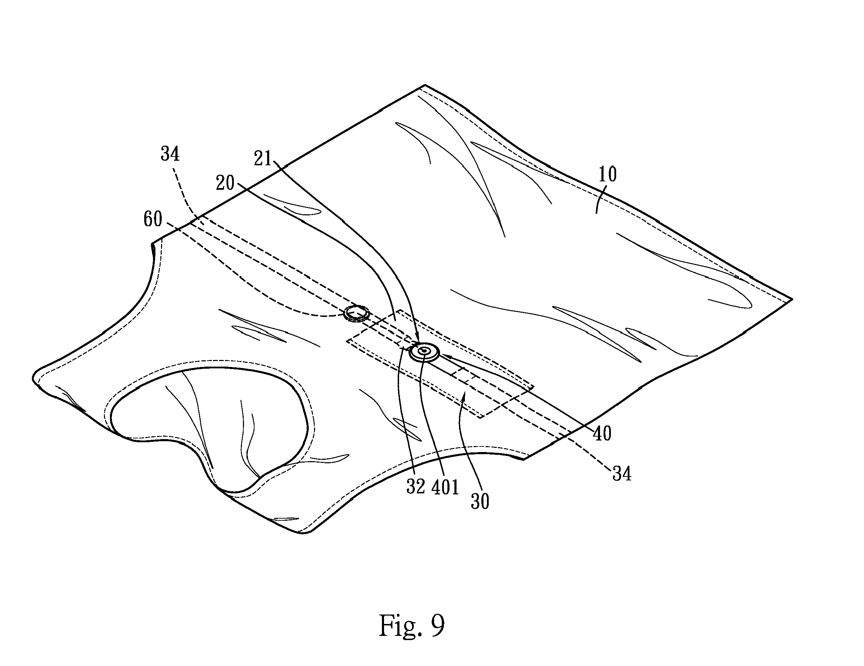

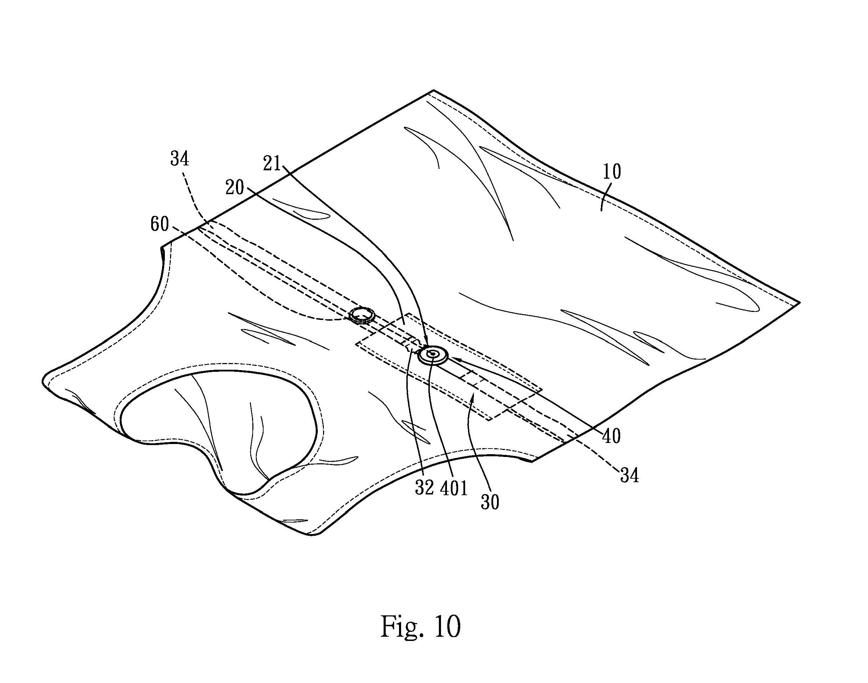

A fourth embodiment of the elastic physiological detection structure according to the present invention comprises a fabric article 10, a main body 20, an elastic member 30, and a detection module 40 and has a structural arrangement that is similar to those of the first, second, and third embodiments with a primary difference being that the elastic member 30 of the first, second, and third embodiments is additionally provided with an extension section 34 and the extension section 34 extends to a sewing line of the fabric article 10, wherein combination can be made with one side sewing line (as shown in Figure 8) or two sewing lines (as shown in Figure 9), or alternatively, the extension section 34 may be arranged to extend completely along a circumference around the fabric article 10 (as shown in Figure 10). Here, the fabric article 10 can be one of a garment, trousers, gloves, an underwear, a vest, a corsage, and a tube-top and in the instant embodiment, a vest is taken as an example of the fabric article 10 for illustration, wherein the fabric article 10 in the form of a vest is wearable on a human body (the fabric article 10 in the form of a vest being shown inside out in the drawings) to establish contact engagement with the surface layer of the human body so as to allow the main body 20 that is combined to the vest of the fabric article 10 to get tight engagement with a specific site of the surface layer of the human body, such as chest or the pit of the stomach, with the detection module 40 that is exposed outside the opening 21 of the main body 20 contacting the surface layer of the human body. The fabric article 10 is formed through weaving or knitting an elastic material or a non-elastic material. When the elastic member 30 comprises an extension section 34, this helps maintain the detection module 40 that is combined to the elastic member 30 and exposed outside the opening 21 of the main body 20 in a flat form and an effect of being retained on the surface layer of the human body can be achieved with the elastic member 30 to allow the detection module 40 that is exposed outside the opening 21 of the main body 20 to attach to a site of the skin where detection is to be made and improve the effect of contact engagement with the surface layer of the human body.

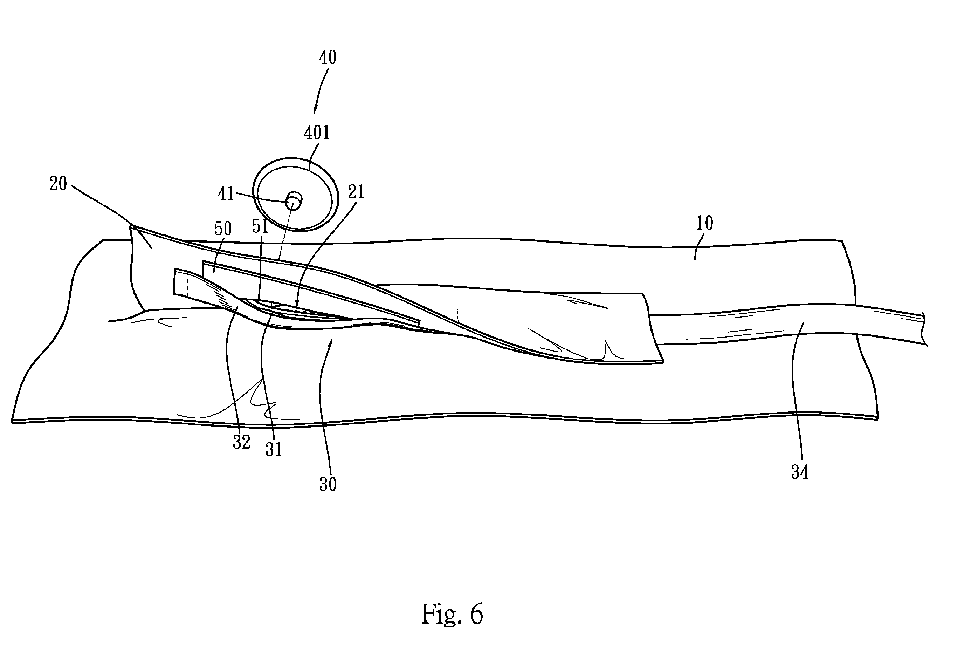

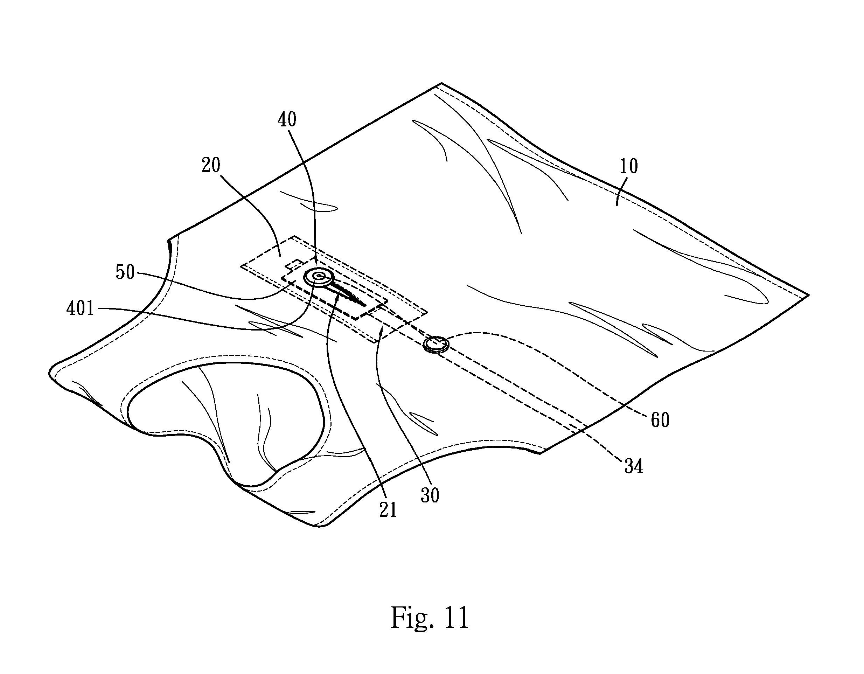

Further, in the above-described first, second, third, and fourth embodiments of the elastic physiological detection structure according to the present invention, the opening 21 of the main body 20 (which in the instant embodiment is illustrated as a single transverse opening 21) may be constructed in such a way that the opening 21 of the main body 20 has an inside surface to which a film layer 50 (as shown in Figures 6 and 11) is coupled or combined. The combination can be achieved with one of sewing, ultrasonic means, thermal fusion, and adhesive and the film layer 50 is provided with an opening 51 corresponding to the opening 21 of the main body 20 so as to allow the detection module 40 that is mounted on the elastic member 30 to extend through the opening 51 of the film layer 50 and the opening 21 of the main body 20 for being exposed outside. The film layer 50 can be one of a plastic piece, a fabric piece (that may be additionally processed to provide stiffness, such as a fabric woven or knitted with acrylic yarns), and a membrane (which can be a plastic membrane, a metal membrane, a carbon membrane, or a membrane made of other materials) in order to improve the stiffness or rigidity of the opening 21 of the main body 20 to allow for easy displacement or sliding of the detection module 40 within the opening 21 of the main body 20 and to prevent the elastic member 30 from being easily pulled out of the opening 21 of the main body 20 so as to prevent the detection module 40 from being separated from a desired displacement trace and thus failing the operation thereof.

Further, in an embodiment of the opening 21 of the main body 20, two film layers (not shown) may be provide and combined with the inside surfaces of the opening 21 of the main body 20. The combination can be achieved with one of sewing, ultrasonic means, thermal fusion, and adhesive. The two film layers are respectively set at left and right sides of the opening 21 of the main body 20 to define a slit (not shown) in such a way that the opening 21 of the main body 20 corresponds to the slit, thereby allowing the detection module 40 that is mounted on the elastic member 30 to extend through the slit of the two film layers and the opening 21 of the main body 20 to get exposed outside. The two film layers can each be one of a plastic piece, a fabric piece (that may be additionally processed to provide stiffness, such as a fabric woven or knitted with acrylic yarns), and a membrane (which can be a plastic membrane, a metal membrane, a carbon membrane, or a membrane made of other materials) in order to improve the stiffness or rigidity of the opening 21 of the main body 20 to allow for easy displacement or sliding of the detection module 40 within the opening 21 of the main body 20 and to prevent the elastic member 30 from being easily pulled out of the opening 21 of the main body 20 so as to prevent the detection module 40 from being separated from a desired displacement trace and thus failing the operation thereof.

Further, in the above-described first, second, third, and fourth embodiments of the elastic physiological detection structure according to the present invention, the detection module 40 can be constructed to comprise, in a first example, an electrode plate 401 (as shown in Figures 1, 3, 7, and 8) for detecting a physiological signal of the surface layer of the human body. The electrode plate 401 is formed by weaving, knitting, or otherwise combining a plurality of non-conductive fiber yarns and a plurality of conductive fiber yarns; or alternatively, the electrode plate 401, in the entirety thereof, is formed solely of a plurality of conductive fiber yarns through weaving, knitting, or other combining means so as to make the entirety of the electrode plate 401 electrically conductive. Further, the plurality of conductive fiber yarns of the electrode plate 401 is woven, knitted, or otherwise combined to form a conductive zone. The conductive zone of the electrode plate 401 is positionable against and thus in tight contact engagement with the skin of the human body in order to detect the physiological signal. The physiological signal can be one of body temperature, heartbeat, pulse, and breath.

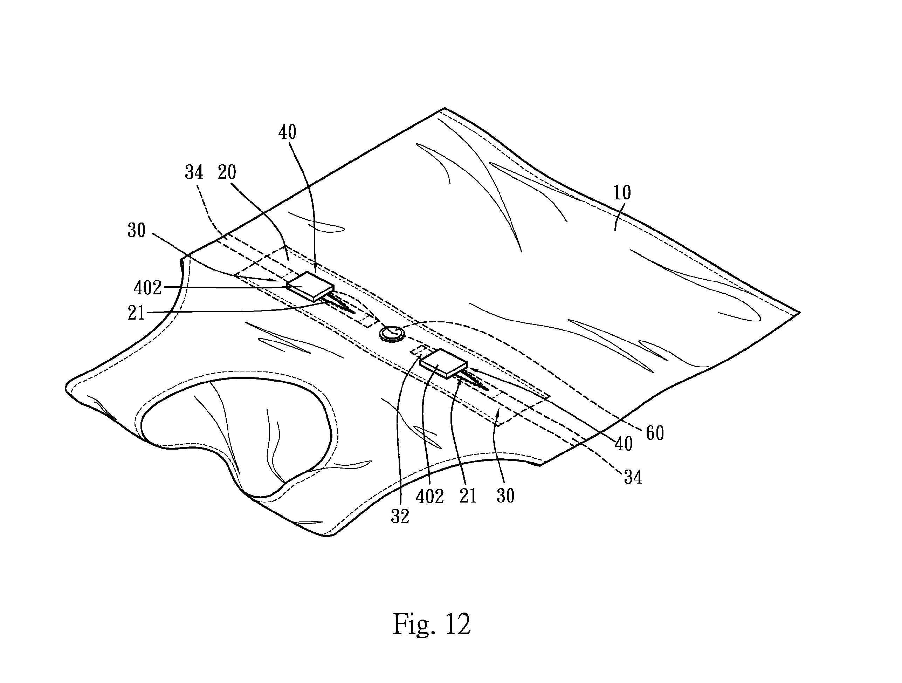

Further, in the above-described first, second, third, and fourth embodiments of the elastic physiological detection structure according to the present invention, the detection module 40 can be constructed to comprise, in a second example, an inclination detection chip and a microcontroller 402 (as shown in Figure 12). The microcontroller 402 is connected to the inclination detection chip so that the microcontroller 402 may detect a variation of the inclination detection chip. The inclination detection chip can be a three-axis acceleration transducer (such as a three-axis low-g micro-machined accelerometer). The three-axis acceleration transducer is operable to detect/calculate inclination angles and accelerations in three axes of X, Y, and Z and the microcontroller 402 is operable to periodically detect and transmit these values. The inclination angle can be used to detect an event of dizziness or fall by setting a change exceeding a threshold of +/-1.0 degree, +/-1.5 degrees, or +/-2.0 degrees. The microcontroller 402 can be set to conduct detection at a fixed time interval with a fixed number of detection operations. For example, abnormal inclination may be identified by means of an average of successively detected values within a period of 30 seconds being determined exceeding +/-1.0 degree (or +/-1.5 degrees or +/-2.0 degrees). Or alternatively, a nine-axis body position transducer (not shown) may be used. The nine-axis body position transducer comprises a three-axis acceleration sensor, three-axis magnetic field sensor, and a three-axis gyro sensor with the range of magnetic field being ±1.3/1.9/2.5/4.0/4.7/5.6/8.1 gausses, the range of the acceleration being ±2g/±4g/±8g, and the range of the gyro being ±250/500/2000 dps. Abnormality can be identified by determining by monitoring and detecting a value of a body position according to the above-mentioned ranges. When the detected value exceeds the ranges, abnormal inclination is identified. When a variation of the inclination or body position exceeds a setting value or a threshold, the microcontroller 402 issues, through a wireless signal transmitter (not shown), a signal.

Further, in the above-described fourth embodiment of the elastic physiological detection structure according to the present invention, the fabric article 10 is provided with a signal transmission terminal 60 (as shown in Figure 12) and the signal transmission terminal 60 is coupled to at least one detection module 40 so that the variation of the physiological signal or the inclination angle of the human body detected by the detection module 40 can be transmitted to the signal transmission terminal 60. In addition, the signal transmission terminal 60 may be coupled to a signal transmitter (not shown) and the signal transmitter comprises a wireless transmission module that transmits, in a wireless manner, the variation of the physiological signal or the inclination angle of the human body to an electronic device (such as a smart mobile phone, a tablet computer, a notebook computer, a desktop computer, and medical facility) or to the cloud for realization of the detection result and to provide preventive medical treatment.

Further, in the above-described first, second, third, and fourth embodiments of the elastic physiological detection structure according to the present invention, a combined assembly of a fabric article 10, a main body 20, an elastic member 30, and a detection module 40 is provided in such a way that the main body 20 is combined with an internal layer of the fabric article 10 and the main body 20 is provided with at least one opening 21 formed therein; the elastic member 30 is combined to a surface of the main body 20; and the detection module 40 is combined to the elastic member 30 and exposed outside the opening 21 of the main body 20, whereby the elastic member 30 helps the detection module 40 to apply an increased force from the fabric article 10 to the surface layer of the human body, allowing the detection module 40 that is located outside the opening 21 of the main body 20 to tightly engage the surface layer of the human body at a site where detection is to be made thereby improving the attachability of the detection module 40 to the surface layer of the human body and enhancing stability of dynamic detection.

Further, the opening 21 of the main body 20 is provided with a predetermined length and the elastic member 30 also comprises a predetermined preserved length 32, so that the detection module 40 is allowed to displace in the opening 21 of the main body 20 by means of the preserved length 32 of the elastic member 30, allowing the detection module 40 to move back and forth in the opening 21 of the main body 20 to provide an effect of changing the site where detection is to be made for detecting physiological signals of different sites of the human body. Further, with such an arrangement, the stability of the detection module 40 can be ensured and the phenomenon of inflaming caused by the detection module 40 being retained on and depressing a fixed location of the skin, which may lead to discomfort, can be eliminated.

Based on the above detailed description, those skilled in the art may appreciate that the present invention can achieve the above-discussed objectives. However, it is noted that the above description is made only to a preferred embodiment of the present invention and is not intending to limit the true scope where the present invention may be put into practice. Thus, simple and equivalent variations and modifications made on the disclosure of the specification and the attached claims are all considered within the scope of the present invention.

## Claims

1. An elastic physiological detection structure, comprising:
a fabric article;
a main body, which is combined to an internal layer of the fabric article, the main body comprising at least one opening formed therein;
an elastic member, which is combined with a surface of the main body; and
a detection module, which is combined with the elastic member and is exposed outside the opening of the detection module to allow the detection module to apply an increased force through the elastic member.

2. The elastic physiological detection structure as claimed in Claim 1, wherein the opening of the main body comprises a longitudinal opening or a transverse opening and the main body comprises one of a piece of fabric, a woven article, and a membrane.

3. The elastic physiological detection structure as claimed in Claim 1 or 2, wherein the opening of the main body has an inside surface to which a film layer is combined, the film layer comprising an opening formed therein to correspond to the opening of the main body, the film layer comprising one of a plastic piece, a fabric piece, and a membrane.

4. The elastic physiological detection structure as claimed in Claim 1 or 2, wherein the opening of the main body has an inside surface to which two film layers are combined in such a way that the two film layers are respectively located at left and right sides of the opening of the main body to define a slit, the opening of the main body corresponding to the slit, the film layers each comprising one of a plastic piece, a fabric piece, and a membrane.

5. The elastic physiological detection structure as claimed in Claim 1, wherein the fabric article comprises one of a garment, trousers, gloves, underwear, a vest, a corsage, and a tube-top and the fabric article is formed through weaving or knitting an elastic material or a non-elastic material.

6. The elastic physiological detection structure as claimed in Claim 1, wherein the opening of the main body has a predetermined length and the elastic member comprises a predetermined preserved length to allow the detection module to selectively displace in the opening of the main body through the preserved length of the elastic member.

7. The elastic physiological detection structure as claimed in Claim 6, wherein the preserved length of the elastic member includes a non-elastic section.

8. The elastic physiological detection structure as claimed in Claim 1, wherein the elastic member further comprises an extension section and the extension section extends to a sewing line of the fabric article.

9. The elastic physiological detection structure as claimed in Claim 1, wherein the combination of the elastic member and the main body is such that two ends of the elastic member and the main body are respectively provided with engageable fastening sections.

10. The elastic physiological detection structure as claimed in Claim 1, wherein the combination comprise one of sewing, ultrasonic means, thermal fusion, and adhesive.

11. The elastic physiological detection structure as claimed in Claim 1, wherein the combination of the detection module and the elastic member is such that mateable coupling sections are respectively provided on the detection module and the elastic member.

12. The elastic physiological detection structure as claimed in Claim 1, wherein the combination of the detection module and the elastic member is such that the detection module is directly mounted to the elastic member.

13. The elastic physiological detection structure as claimed in Claim 1, wherein the detection module comprises an electrode plate that is adapted to detect a physiological signal of a surface layer of a human body and the electrode plate is connected to a signal transmission terminal.

14. The elastic physiological detection structure as claimed in Claim 1, wherein the detection module comprises an inclination detection chip and a microcontroller, the microcontroller being connected to the inclination detection chip so that the microcontroller detects a variation of the inclination detection chip.
